(19) Europäisches Patentamt
European Patent Office
Office européen des brevets

(11) Veröffentlichungsnummer: **0 092 674**
**B1**

(12) # EUROPÄISCHE PATENTSCHRIFT

(45) Veröffentlichungstag der Patentschrift: **06.11.85**

(51) Int. Cl.[4]: **C 07 D 249/08,** A 01 N 43/653 // C07C49/255

(21) Anmeldenummer: **83102764.4**

(22) Anmeldetag: **21.03.83**

(54) **Triazolverbindungen, Verfahren zu ihrer Herstellung und diese enthaltende Fungizide.**

(30) Priorität: **31.03.82 DE 3211850**

(43) Veröffentlichungstag der Anmeldung: **02.11.83 Patentblatt 83/44**

(45) Bekanntmachung des Hinweises auf die Patenterteilung: **06.11.85 Patentblatt 85/45**

(84) Benannte Vertragsstaaten: **AT BE CH DE FR GB IT LI NL SE**

(56) Entgegenhaltungen:
**EP - A - 0 039 405**
**EP - A - 0 040 350**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Zeeh, Bernd, Dr., Thorwaldsenstrasse 5, D-6700 Ludwigshafen (DE)**
Erfinder: **Meyer, Norbert, Dr., Stahlbuehlring 155A, D-6802 Ladenburg (DE)**
Erfinder: **Sauter, Hubert, Dr., Neckarpromenade, D-6800 Mannheim (DE)**
Erfinder: **Pommer, Ernst-Heinrich, Dr., Berliner Platz 7, D-6703 Limburgerhof (DE)**
Erfinder: **Ammermann, Eberhard, Dr., Sachsenstrasse 3, D-6700 Ludwigshafen (DE)**

**EP 0 092 674 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue, wertvolle Triazolverbindungen, Verfahren zu ihrer Herstellung, diese Verbindungen enthaltende fungizide Mittel und ihre Verwendung zur Bekämpfung von Pilzen.

Es ist bekannt, Triazolverbindungen z.B. das 5,5-Dimethyl-1-(4-chlorphenyl)-3-(1,2,4-triazolyl-1)-2-oxa-hexan-4-on als Fungizid zu verwenden (EP-A-39405). Seine Wirkung ist jedoch unbefriedigend.

Es ist ferner bekannt, Azolverbindungen, die den Phenoxyrest, den tertiär-Butylrest und einen Triazolrest enthalten, als Fungizide zu verwenden (EP-A-40350). Die Wirkung dieser Verbindungen ist aber unbefriedigend und es besteht die Aufgabe, Verbindungen mit besserer Wirkung zu entwickeln.

Es wurde nun gefunden, dass Triazolverbindungen der Formel (I)

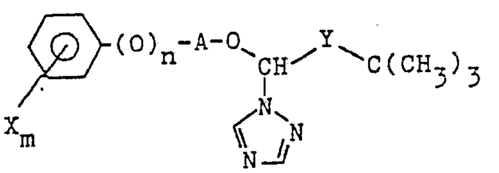 (I)

in welcher

X für Halogen, $C_1$-$C_4$-Alkyl, $C_1$-$C_4$-Alkoxy-, Trifluormethyl oder Phenyl steht und

m eine ganze Zahl von 0 bis 5 ist, wobei die einzelnen Gruppen X gleich oder verschieden sind, wenn m grösser als 1 ist,

Y für CO oder die Gruppe $CR^1OR^2$ steht, in welchen $R^1$ Wasserstoff oder $C_1$-$C_4$-Alkyl bedeutet und $R^2$ Wasserstoff, $C_1$-$C_4$-Alkyl, $C_2$-$C_4$-Alkenyl, $C_2$-$C_4$-Alkinyl bedeutet,

A für eine Alkylenkette mit 2 bis 6 Kohlenstoffatomen steht, die durch eine oder mehrere Alkylgruppen mit 1 bis 2 Kohlenstoffatomen substituiert sein kann, und

n 0 oder 1 bedeutet,

sowie deren Säureadditionssalze und Metallkomplexsalze eine starke fungizide Wirkung haben.

In den neuen Verbindungen der Formel (I) ist das azolylsubstituierte Kohlenstoffatom chiral; die Wirkstoffe fallen demgemäss als Enantiomerengemische an, die in die optisch aktiven Verbindungen getrennt werden können. Im Falle der Alkohole und Ether ($Y=CR^1OR^2$) treten durch das zusätzliche chirale Zentrum Diastereomerengemische auf, die in üblicher Weise, z.B. durch Kristallisation oder Chromatographie in die einzelnen Komponenten getrennt werden können. Alle genannten Verbindungen und ihre Anwendung als Fungizide werden von der vorliegenden Erfindung umfasst. Für die Anwendung der neuen Verbindungen als Fungizide ist jedoch eine Trennung der Enantiomeren oder Diastereomeren normalerweise nicht erforderlich.

Der Phenylrest kann beispielsweise folgendermassen durch $X_m$ substituiert sein:

2-Fluor-, 4-Fluor-, 2-Chlor-, 3-Chlor-, 4-Chlor-, 4-Brom-, 2,4-Dichlor-, 2,4,6-Trichlor-, 3,5-Dichlor-, 2-Chlor-4-phenyl-, 2-Methyl-4-chlor-, 2-Methyl-, 3-Methyl-, 4-Methyl-, 3-tert.-Butyl-, 4-tert.-Butyl-, 2-Methoxy-, 3-Methoxy-, 4-Methoxy-, 3,5-Dimethoxy-, 3-n-Butoxy-, 4-n-Butoxy-, 2-Methoxy-4-methyl-, 3-Trifluormethyl-, $R^1$ bedeutet beispielsweise Wasserstoff, Methyl, Ethyl, n-Propyl, Isopropyl, n-Butyl.

$R^2$ bedeutet beispielsweise Wasserstoff, Methyl, Ethyl, n-Propyl, Prop-2-enyl-(1), Prop-2-inyl-(1), n-Butyl, 2-Methylprop-2-enyl-(1).

A bedeutet beispielsweise Ethylen, Propylen, Butylen, Pentylen, Hexylen, Methylpropylen, Methylbutylen, Dimethylbutylen, Methylpentylen, Dimethylpentylen, Trimethylpentylen, Methylhexylen, Dimethylhexylen, Trimethylhexylen, Ethylpentylen, Ethylhexylen, Methylethylhexylen.

Geeignete Säureadditionssalze sind beispielsweise die Chloride, Bromide, Sulfate, Nitrate, Phosphate, Azetate, Oxalate oder Dodecylbenzolsulfonate. Die Wirksamkeit der Salze geht auf das Kation zurück, so dass die Wahl des Anions beliebig ist. Nichtphytotoxische Anionen werden bevorzugt.

Geeignete Metallkomplexe sind Verbindungen der Formel

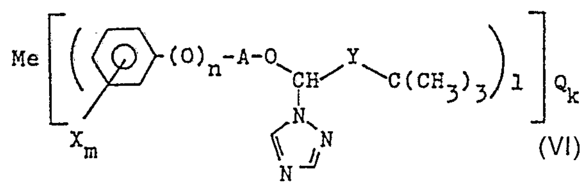 (VI)

in der

$X_m$, A, Y und n die oben angegebene Bedeutung haben und Me ein Metall, z.B. Kupfer, Zink, Zinn, Mangan, Eisen, Cobalt oder Nickel bedeutet,

Q für das Anion einer anorganischen Säure steht, z.B. Salzsäure, Schwefelsäure, Phosphorsäure oder Bromwasserstoffsäure und

1 und K 1, 2, 3 oder 4 bedeuten.

Die neuen Verbindungen mit Y=CO können beispielsweise hergestellt werden, indem man

a) Halogenetherketone der Formel (II)

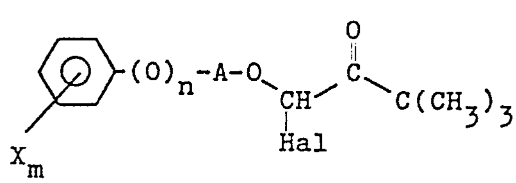 (II)

in welcher $X_m$, A und n die oben angegebene Bedeutung haben und

Hal für Chlor oder Brom steht, mit 1,2,4-Triazol in Gegenwart eines Säurebindemittels und gegebenenfalls in Gegenwart eines Verdünnungsmittels umsetzt.

Die Halogenetherketone der Formel (II) können nach bekannten Verfahren hergestellt werden, indem man z.B. in Ketonen der Formel

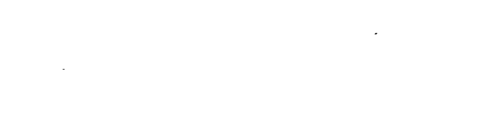 (VII)

in welcher

$X_m$, A und n die oben angegebene Bedeutung haben, eines der beiden aktiven Wasserstoffatome in üblicher Weise gegen Chlor oder Brom austauscht. Die entstehenden Halogenetherketone der Formel (II) können direkt weiter umgesetzt werden.

Die Ether der Formel (VII) können nach bekannten Verfahren erhalten werden, indem man z.B. Alkohole der Formel (IV)

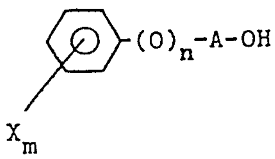

(IV)

mit Chlor(Brom)pinakolin der Formel

$$(Br)Cl-CH_2-CO-C(CH_3)_3 \qquad (VIII)$$

in Gegenwart einer starken Base, wie z.B. Natriumhydrid, und in Gegenwart eines inerten organischen Lösungsmittels, wie z.B. Tetrahydrofuran oder Dimethylformamid, bei Temperaturen zwischen 20 und 100° C umsetzt; oder indem man Halogenide der Formel

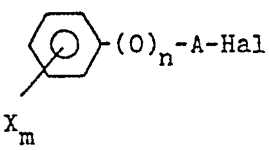

(IX)

in welcher $X_m$, A, Hal und n die oben angegebene Bedeutung haben, mit Hydroxypinakolin der Formel

$$HO-CH_3-CO-C(CH_3)_3$$

in einem Zweiphasensystem, wie z.B. wässerige Natron- oder Kalilauge/Toluol oder Methylenchlorid, unter Zusatz eines Phasentransfer-Katalysators, wie beispielsweise Ammoniumverbindungen wie Triethyl-benzyl-ammoniumchlorid, umsetzt.

Die neuen Verbindungen mit Y=CO können beispielsweise auch hergestellt werden, indem man

b) Triazolylhalogenketone der Formel

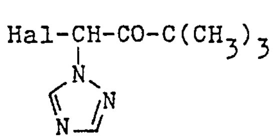

(III)

in welcher

Hal die oben angegebene Bedeutung hat, mit Alkoholen der Formel

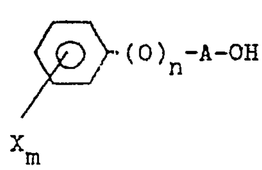

(IV)

in welcher

X, A, m und n die oben angegebene Bedeutung haben, in Gegenwart eines Säurebindemittels und gegebenenfalls in Gegenwart eines Verdünnungsmittels umsetzt.

Die als Ausgangsstoffe für das Verfahren (b) zu verwendenden Triazolylhalogenketone sind durch die Formel (III) allgemein definiert und bekannt (vergleiche DE-OS 2756269). Sie werden erhalten, indem man Chlor(Brom)pinakolin der Formel (III) mit 1,2,4-Triazol in Gegenwart eines Säurebindemittels, wie z.B. Kaliumcarbonat, und in Gegenwart eines inerten organischen Lösungsmittels, wie z.B. Aceton, bei Temperaturen zwischen 60 und 120°C umsetzt. Eines der beiden aktiven Wasserstoffatome wird anschliessend in üblicher Weise gegen Chlor oder Brom ausgetauscht.

Die ausserdem für das Verfahren (b) als Ausgangsstoffe zu verwendenden Alkohole sind durch die Formel (IV) allgemein definiert. In dieser Formel stehen $X_m$, A und n vorzugsweise für die Reste, die bereits im Zusammenhang mit der Beschreibung der neuen Stoffe der Formel (I) vorzugsweise für diese Substituenten genannt wurden.

Für die Umsetzung gemäss Verfahrensvarianten (a) und (b) kommen als Verdünnungsmittel inerte organische Lösungsmittel in Frage. Hierzu gehören vorzugsweise Ketone, wie Diethylketon, insbesondere Aceton und Methylethylketon; Nitrile, wie Propionitril, insbesondere Acetonitril; Alkohole, wie Ethanol oder Isopropanol; Ether, wie Tetrahydrofuran oder Dioxan; Benzol; Formamide; wie insbesondere Dimethylformamid; und halogenierte Kohlenstoffatome.

Die Umsetzungen nach Verfahren (a) und (b) werden in Gegenwart eines Säurebinders vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat oder wie Silbercarbonat, oder wie niedere Alkylamine, Cycloalkylamine oder Aralkylamine, beispielsweise Triethylamin, Dimethylbenzylamin; oder wie Pyridin und Diazabicyclooctan.

Bei der Verfahrensvariante (a) kann auch ein entsprechender Überschuss an Triazol verwendet werden.

Die Reaktionstemperaturen können bei den Verfahren (a) und (b) in einem grösseren Bereich variiert werden. Im allgemeinen arbeitet man zwischen etwa 20 bis etwa 150°C.

Bei Anwesenheit eines Lösungsmittels wird zweckmässigerweise beim Siedepunkt des jeweiligen Lösungsmittels gearbeitet.

Bei der Durchführung des Verfahrens (a) bzw. (b) setzt man auf 1 mol der Verbindung der Formel (II) bzw. (III) vorzugsweise 1 bis 2 mol 1,2,4-Triazol bzw. 1 bis 2 mol Alkohol der Formel (IV) und jeweils 1 bis 2 mol Säurebinder ein.

Die neuen Verbindungen mit Y=CHOH können beispielsweise auch hergestellt werden, indem man

c) die Ketone der Formel (I), in denen R eine CO-Gruppe ist, einer Reduktion unterwirft, z.B. durch Einwirkung von komplexen Hydriden, bevorzugt Natriumborhydrid in Gegenwart eines polaren Lösungsmittels, z.B. eines Alkohols, bevorzugt Methanol oder Ethanol bei Temperaturen zwischen 0 und 100°C und anschliessender Hydrolyse mit wässerigen Basen oder Säuren oder durch Einwirkung von Wasserstoff in Gegenwart eines Hydrierungskatalysators wie Platin oder Raney-Nickel und in Gegenwart eines polaren Lösungsmittels wie Methanol, Ethanol oder Ethylacetat bei Temperaturen zwischen 20 und 100°C und Drücken von 1 bis 100 bar.

Die neuen Verbindungen $Y=CR^1OH$ mit $R^1$ verschieden von Wasserstoff können beispielsweise hergestellt werden, indem man Ketone der Formel (I) (Y=CO) mit 0,8 bis 1,2 Äquivalenten einer Grignardverbindung der Formel (V)

$$R^1-MgHal \qquad (V)$$

in der $R^1$ $C_1$-$C_4$-Alkyl und Hal Chlor, Brom oder Iod bedeutet, vorzugsweise in Anwesenheit eines Lösungsmittels und gegebenenfalls in Anwesenheit eines ausbeutesteigernden Salzes umsetzt.

Als Lösungsmittel kommen bevorzugt Ether in Frage, wie Diethylether, Di-n-propylether, Tetrahydrofuran oder Anisol, ferner tertiäre Amine wie N,N-Diethylanilin sowie Phosphorsäure-tris(dimethylamid); gegebenenfalls kann man die Reaktion auch im Gemisch dieser Lösungsmittel mit aliphatischen oder aromatischen Kohlenstoffatomen wie n-Hexan oder Toluol durchführen. Als ausbeutesteigernde Salze, die die üblichen Nebenreaktionen unterdrücken, kommen insbesondere wasserfreie Magnesiumhalogenide wie wasserfreies Magnesiumbromid oder wasserfreie Tetraalkylammoniumhalogenide, wie z.B. Tetra-n-butylammoniumchlorid in Frage. Die Reaktionstemperaturen lassen sich je nach Lösungsmittel zwischen 0 und 100°C variieren, bevorzugt sind Temperaturen zwischen 0 und 60°C. Die hierbei primär entstandenen Magnesiumalkoholate werden sodann durch Hydrolyse mit verdünnten wässerigen Säuren, wie Salzsäure, Schwefelsäure oder bevorzugt Essigsäure oder besonders bevorzugt mit wässeriger Ammoniumchloridlösung in die Alkohole übergeführt, und diese nach Entfernen der wässerigen Phase, falls gewünscht, in üblicher Weise durch Extraktion, Umkristallisieren oder Chromatographie gereinigt.

Die neuen Verbindungen mit $Y=CR^1OR^2$ mit $R^2$ verschieden von Wasserstoff können beispielsweise hergestellt werden, indem man die sekundären oder tertiären Alkohole der Formel (I)

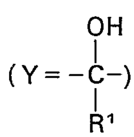

oder ihr Alkali- oder quartären Ammoniumsalze mit einem Alkylierungsmittel der Formel (X)

$$L-R^2 \qquad (X)$$

in der $R^2$ die oben genannten Bedeutungen ausser Wasserstoff hat und L Chlor oder Brom oder den Rest des Monoalkyl-schwefelsäureesters Alkyl $-OSO_2-O-$ bedeutet, gegebenenfalls in Gegenwart eines Lösungs- oder Verdünnungsmittels gegebenenfalls in Gegenwart anorganischer oder organischer Basen und/oder eines Reaktionsbeschleunigers zwischen 0 und 100°C umsetzt.

Geeignete anorganische oder organische Basen, die gegebenenfalls auch als säurebindende Mittel in die Reaktion eingesetzt werden können, sind beispielsweise Alkali- und Erdalkalihydroxide wie Natriumhydroxid, Kalimhydroxid, Calciumhydroxid, Alkalicarbonate wie Kalium- oder Natriumcarbonate, Alkalihydride wie Natriumhydrid, Alkali- oder Erdalkalialkoholate wie Natriummethylat, Magnesiummethylat oder Natriumisopropylat oder tertiäre Amine wie Trimethylamin, Triethylamin, N,N-Dimethylanilin, N,N-Dimethylcyclohexylamin, N-Methylpiperidin oder Pyridin. Es können aber auch andere übliche Basen verwendet werden.

Mit geeigneten Basen wie z.B. Alkalihydrid wie Natriumhydrid oder Lithiumalkylen wie Butyllithium oder mit Alkali- oder Erdalkalialkoholaten wie Natriummethylat können die Alkohole der Formel (II) auch in einer vorgeschalteten Umsetzung zunächst in ihre Alkoholat-Salze überführt werden und dann als solche zur Reaktion gebracht werden.

Zu den bevorzugten Lösungs- bzw. Verdünnungsmitteln gehören Halogenkohlenwasserstoffe wie beispielsweise Methylenchlorid, Chloroform, 1,2-Dichlorethan, Chlorbenzol; aliphatische oder aromatische Kohlenwasserstoffe wie Cyclohexan, Petrolether, Benzol, Toluol oder Xylole, Ester wie Essigsäureethylester, Amide wie Dimethylformamid, Nitrile wie Acetonitril, Sulfoxide wie Dimethylsulfoxid, Ketone wie Aceton oder Methylethylketon, Ether wie Diethylether, Tetrahydrofuran oder Dioxan oder entsprechende Gemische.

Als Reaktionsbeschleuniger kommen vorzugsweise Metallhalogenide wie Kaliumiodid, Kronenether, quartäre Ammoniumverbindungen wie Tetrabutylammoniumiodid oder Säuren oder Kombinationen dieser Reaktionsbeschleuniger in Frage.

Die Umsetzungen werden im allgemeinen bei Temperaturen zwischen 0 und 100°C in einem Zeitraum von 1 bis 60 h durchgeführt, drucklos oder unter Druck, kontinuierlich oder diskontinuierlich.

Zur Isolierung der neuen Verbindungen wird nach üblichen Methoden vorgegangen. Im allgemeinen bedürfen die anfallenden Produkte keiner weiteren Reinigung, sie können aber nach bekannten Methoden wie Umkristallisation, Extraktion, Destillation oder Chromatographie weiter gereinigt werden. Falls gewünscht, können die neuen Verbindungen der Formel (I) auch in Salze mit anorganischen oder organischen Säuren übergeführt werden, wie beispielsweise in Salze der Salzsäure, Bromwasserstoffsäure, Salpetersäure,

Oxalsäure, Essigsäure, Schwefelsäure, Phosphorsäure oder Dodecylbenzolsulfonsäure.

Ferner lassen sich die Verbindungen der Formel (I) nach bekannten Methoden in Metallkomplexe überführen. Das kann durch Umsetzung dieser Verbindungen mit geeigneten Metallsalzen wie beispielsweise Kupfer(II)-chlorid, Zink(II)-chlorid, Eisen(III)-chlorid, Kupfer(II)-nitrat, Mangan-(II)-chlorid oder Nickel(II)-bromid erfolgen.

Die Herstellung der neuen Verbindungen der Formel (I) wird durch folgende Beispiele erläutert:

*Beispiel 1:*

57,8 g 4-Chlor-6,6-dimethyl-1-(4-chlorphenyl)-3-oxa-heptan-5-on gelöst in 300 ml Acetonitril wurden bei Raumtemperatur unter Rühren mit 27,6 g 1,2,4-Triazol versetzt. Man liess 17 h bei 25°C rühren, engte durch Abdestillieren des Lösungsmittels ein und nahm den Rückstand mit Methylenchlorid/Wasser auf. Die organische Phase wurde abgetrennt, nochmals mit Wasser gewaschen, über Natriumsulfat getrocknet und eingeengt. Nach einer säulenchromatographischen Trennung erhielt man 31,5 g (48,9% d.Th.) 6,6-Dimethyl-1-(4-chlorphenyl)-4-(1,2,4-triazol-1-yl)-3-oxa-heptan-5-on als viskoses Öl (Verbindung Nr. 4).

IR (Film): 2978, 2942, 1728, 1502, 1494, 1276, 1136, 1113, 1104, 1069, 1016 $cm^{-1}$.

*Herstellung des Vorproduktes*

Unter Stickstoff wurden 3,6 g 80%iges Natriumhydrid in 150 ml trockenem Tetrahydrofuran suspendiert. Man tropfte 15,7 g 4-Chlorphenylethanol in 50 ml trockenem Tetrahydrofuran zu, liess 2 h bei 25°C nachrühren und tropfte dann bei −15 bis −20°C 19,7 g Brompinakolin zu. Nach 5 h Rühren bei 25°C wurde mit 200 ml Wasser hydriert, dreimal mit je 100 ml Methyl-tert.-butylether extrahiert und der vereinigte Extrakt dreimal mit je 50 ml Wasser gewaschen. Die getrocknete organische Phase wurde destilliert. Dabei erhielt man 7,5 g (71% d.Th.) 6,6-Dimethyl-1-(4-chlorphenyl)-3-oxa-heptan-5-on (0,3 mbar/124 bis 154°C).

57,3 g 6,6-Dimethyl-1-(4-chlorphenyl)-3-oxa-heptan-5-on wurden in 200 ml Methylenchlorid gelöst und bei leicht exothermer Reaktion tropfenweise mit 33,4 g (0,25 mol) Sulfurylchlorid versetzt. Man rührte 17 h bei 25°C nach, hydrolysierte mit Eiswasser, wusch die organische Phase mit 10%iger Natriumbicarbonatlösung und anschliessend mit Wasser bis zur neutralen Reaktion. Nach Trocknen der Methylenchloridphase über Natriumsulfat wurde eingeengt und im Vakuum bei 0,1 mbar von Lösungsmittelresten befreit. Man erhielt 59,5 g (91,4% d.Th.) 4-Chlor-6,6-dimethyl-1-(4-chlorphenyl)-3-oxa-heptan-5-on.

*Beispiel 2:*

Zu 16,1 g 6,6-Dimethyl-1-(4-chlorphenyl)-4-(1,2,4-triazol-1-yl)-3-oxa-heptan-5-on gelöst in 100 ml Methanol gab man bei Raumtemperatur unter Rühren 1,1 g (0,03 mol) Natriumborhydrid. Nach Abklingen der exothermen Reaktion erhitzte man das Reaktionsgemisch 1 h unter Rückfluss. Danach wurde eingeengt und der Rückstand mit Methylenchlorid und Wasser geschüttelt. Die organische Phase wurde abgetrennt, mit Wasser gewaschen, über Natriumsulfat getrocknet und eingeengt. Nach Entfernung von Lösungsmittelresten im Vakuum bei 0,1 mbar erhielt man 15,3 g (95,8% d.Th.) 6,6-Dimethyl-1-(4-chlorphenyl)-4-(1,2,4-triazol-1-yl)-3-oxa-heptan-5-ol (Verbindung Nr. 5).

IR (Film): 3270, 2958, 2872, 1494, 1275, 1133, 1109, 1093, 1015 $cm^{-1}$.

*Beispiel 3:*

Zu einer unter Stickstoff gerührten Suspension von 1,6 g Natriumhydrid in 100 ml trockenem Tetrahydrofuran tropfte man bei Raumtemperatur 17 g 6,6-Dimethyl-1-(4-chlorphenoxy)-4-(1,2,4-triazol-1-yl)-3-oxa-heptan-5-ol in 50 ml trockenem Tetrahydrofuran, rührte 3 h nach, fügte 8,5 g (0,06 mol) Methyliodit zu und liess weitere 5 h bei 25°C rühren. Nach Zugabe von 150 ml Wasser wurde dreimal mit je 100 ml Methyl-tert.-butylether extrahiert, die organische Phase dreimal mit je 50 ml Wasser gewaschen, über Natriumsulfat getrocknet und im Vakuum bei 0,1 mbar eingeengt. Dabei erhielt man 15,0 g (84,7% d.Th.) 6,6-Dimethyl-5-methoxy-1-(4-chlorphenoxy)-4-(1,2,4-triazol-1-yl)-3-oxa-heptan (Verbindung Nr. 29).

IR (Film): 2957, 1493, 1455, 1275, 1247, 1133, 1116, 1095, 1065 $cm^{-1}$.

Nach entsprechendem Verfahren wurden die folgenden Verbindungen der allgemeinen Formel (I) hergestellt:

*Tabellen →*

Die neuen Verbindungen und ihre Salze und Metallkomplexverbindungen zeichnen sich durch eine hervorragende Wirksamkeit gegen ein breites Spektrum von pflanzenpathogenen Pilzen, insbesondere aus der Klasse der Ascomyzeten und Basidiomyzeten, aus. Sie sind zum Teil systemisch wirksam und können als Blatt- und Bodenfungizide eingesetzt werden. Ferner können sie auch im Materialschutz verwendet werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Bananen, Erdnüsse, Zuckerrohr, Obst und Zierpflanzen im Gartenbau sowie Gemüse – wie Gurken, Bohnen und Kürbisgewächse.

Die neuen Verbindungen sind insbesondere geeignet zur Bekämpfung folgender Pflanzenkrankheiten:

Erysiphe graminis (echter Mehltau) in Getreide,
Erysiphe cichoracearum (echter Mehltau) an Kürbisgewächsen
Podosphaera leucotricha an Äpfeln,
Uncinula necator an Reben,
Erysiphe polygoni an Bohnen,
Sphaerotheca pannosa an Rosen,
Puccinia-Arten an Getreide,

*Tabelle 1*

| Verbindung Nr. | $X_m$ | n | A | Y | IR (Film) [$cm^{-1}$] |
|---|---|---|---|---|---|
| 1 | — | 1 | $-(CH_2)_2-$ | C=O | 3135, 2960, 2865, 1718, 1593, 1582, 1490, 1273, 1245, 1132, 1062 |
| 2 | — | 1 | $-(CH_2)_2-$ | CHOH | 3290, 2945, 2865, 1596, 1584, 1489, 1450, 1362, 1272, 1240, 1188, 1130, 1078 |
| 3 | — | 0 | $-(CH_2)_3-$ | C=O | 3020, 2978, 2928, 2885, 1720, 1494, 1273, 1194, 1131, 1108, 1062 |
| 4 | 4-Cl | 0 | $-(CH_2)_2-$ | C=O | 2978, 2942, 1728, 1502, 1494, 1276, 1136, 1113, 1104, 1069, 1016 |
| 5 | 4-Cl | 0 | $-(CH_2)_2-$ | CHOH | 3270, 2958, 2873, 1494, 1275, 1133, 1109, 1093, 1015 |
| 6 | 4-Cl | 1 | $-(CH_2)_2-$ | C=O | 2975, 1728, 1493, 1277, 1248, 1137, 1104, 1095, 1067, 826 |
| 7 | — | 0 | $-CH_2CH(CH_3)CH_2CH(CH_3)CH_2-$ | C=O | 2863, 2926, 1729, 1502, 1276, 1135, 1100, 1066 |
| 8 | 2,4-$Cl_2$ | 1 | $-(CH_2)_2-$ | C=O | 2975, 1728, 1502, 1483, 1453, 1282, 1276, 1258, 1137, 1104, 1068 |
| 9 | 2,4-$Cl_2$ | 1 | $-(CH_2)_2-$ | CHOH | 3210, 3150, 2963, 1507, 1484, 1452, 1292, 1279, 1266, 1133, 1115, 1087, 1081 |
| 10 | 2,4,6-$Cl_3$ | 1 | $-(CH_2)_2-$ | C=O | 2773, 1720, 1504, 1466, 1446, 1262, 1135, 1127, 1105, 1046 |
| 11 | 2,4,6-$Cl_3$ | 1 | $-(CH_2)_2-$ | CHOH | 3260, 2948, 2862, 1548, 1496, 1460, 1445, 1363, 1273, 1258, 1190, 1130 |
| 12 | — | 0 | $-(CH_2)_2-CH(CH_3)-$ | C=O | 2965, 2926, 2864, 1722, 1496, 1366, 1273, 1130, 1096, 1058 |
| 13 | — | 0 | $-(CH_2)_2-CH(CH_3)-$ | CHOH | 3340, 2948, 2863, 1493, 1448, 1363, 1272, 1187, 1128, 1070, 1018 |
| 14 | — | 1 | $-(CH_3)_3-$ | C=O | 2967, 1727, 1498, 1478, 1276, 1246, 1135, 1094, 1065 |
| 15 | — | 1 | $-(CH_3)_3-$ | CHOH | 3300, 2956, 2873, 1600, 1499, 1473, 1275, 1245, 1132, 1081 |
| 16 | — | 0 | $-(CH_2)_2-$ | C=O | 2970, 1727, 1501, 1276, 1135, 1109, 1068, 1002 |
| 17 | — | 0 | $-(CH_2)_2-$ | CHOH | 3270, 1499, 1275, 1132, 1111, 1097, 1080 |
| 22 | 2,6-$Cl_2$ | 1 | $-(CH_2)_2-$ | C=O | 2975, 2960, 2878, 1728, 1502, 1460, 1441, 1276, 1253, 1136, 1069 |
| 23 | 2,6-$Cl_2$ | 1 | $-(CH_2)_2-$ | CHOH | 3280, 2956, 1459, 1441, 1275, 1253, 1133, 1114, 1070 |
| 25 | — | 0 | $-(CH_2)_4-$ | C=O | 2958, 1728, 1501, 1479, 1275, 1134, 1110, 1067 |
| 28 | 4-Cl | 1 | $-(CH_2)_2-$ | CHOH | 3300, 2957, 2873, 1493, 1455, 1277, 1247, 1172, 1134, 1093 |
| 29 | 4-Cl | 1 | $-(CH_2)_2-$ | $CHOCH_3$ | |
| 30 | 4-Cl | 1 | $-(CH_2)_2-$ | $CH(OCH_2CH=CH_2)$ | 2958, 2872, 1493, 1275, 1247, 1133, 1093, 1006 |

Nach entsprechenden Verfahren können die folgenden Verbindungen der allgemeinen Formel (I) hergestellt werden:

*Tabelle 2*

| Verbindung Nr. | $X_m$ | n | A | Y | IR (Film) [$cm^{-1}$] |
|---|---|---|---|---|---|
| 18 | 3-Cl | 0 | $-(CH_2)_2-$ | C=O | |
| 19 | 3-Cl | 0 | $-(CH_2)_2-$ | CHOH | |
| 20 | 2-Cl | 0 | $-(CH_2)_2-$ | C=O | |
| 21 | 2-Cl | 0 | $-(CH_2)_2-$ | CHOH | |
| 24 | 2,6-$Cl_2$ | 1 | $-(CH_2)_2-$ | $C(CH_3)OH$ | |
| 26 | — | 0 | $-CH(CH_3)CH_2-$ | C=O | |
| 27 | — | 0 | $-CH(CH_3)CH_2-$ | CHOH | |

Rhizoctonia solani an Baumwolle sowie
Helminthosporium-Arten an Getreide,
Ustilago-Arten an Getreide und Zuckerrohr,
Rhynchosporium secale an Getreide,
Venturia inaequalis (Apfelschorf).

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwekken; sie sollten in jedem Fall eine feine und gleichmässige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen je nach der Art des gewünschten Effektes zwischen 0,1 und 3 kg Wirkstoff oder mehr je ha. Die neuen Verbindungen können auch in Materialschutz eingesetzt werden. Bei der Anwendung der Wirkstoffe im Materialschutz, z.B. als Fungizide für Anstrichfarben und Weich-Polyvinylchlorid, betragen die Aufwandmengen 0,05 bis 5% (Gew.-%) Wirkstoff, bezogen auf das Gesamtgewicht der zu konservierenden Farben bzw. des mikrozid auszurüstenden Polyvinylchlorids. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, dass man das Holz mit diesen Mitteln behandelt, beispielsweise tränkt oder anstreicht.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Giessen.

Beispiele für solche Zubereitungen sind:

I. Man vermischt 90 Gewichtsteile der Verbindung des Beispiels 1 mit 10 Gewichtsteilen N-Methyl-alpha-pyrrolidon und erhält eine Lösung, die zur Anwendung in Form kleinster Tropfen geeignet ist.

II. 10 Gewichtsteile der Verbindung des Beispiels 2 werden in einer Mischung gelöst, die aus 90 Gewichsteilen Xylol, 6 Gewichtsteilen des Anlagerungsproduktes von 8 bis 10 mol Ölsäure-N-monoethanolamid, 2 Gewichtsteilen Calciumsalz der Dodecylbenzolsulfonsäure und 2 Gewichtsteilen des Anlagerungsproduktes von 40 mol Ethylenoxid an 1 mol Ricinusöl besteht. Durch feines Verteilen der Mischung in Wasser erhält man eine wässerige Dispersion.

III. 20 Gewichtsteile der Verbindung des Beispiels 3 werden in einer Mischung gelöst, die aus 60 Gewichtsteilen Cyclohexanon, 30 Gewichtsteilen Isobutanol, 10 Gewichtsteilen des Anlagerungsproduktes von 40 mol Ethylenoxid an 1 mol Ricinusöl besteht. Durch Eingiessen und feines Verteilen der Lösung in Wasser erhält man eine wässeige Dispersion.

IV. 20 Gewichtsteile der Verbindung des Beispiels 1 werden in einer Mischung gelöst, die aus 25 Gewichtsteilen Cyclohexanon, 65 Gewichtsteilen einer Mineralölfraktion vom Siedepunkt 210 bis 280° C und 10 Gewichtsteilen des Anlagerungsproduktes von 40 mol Ethylenoxid an 1 mol Ricinusöl besteht. Durch Eingiessen und feines Verteilen der Lösung in Wasser erhält man eine wässerige Dispersion.

V. 80 Gewichtsteile der Verbindung des Beispiels 1 werden mit 3 Gewichtsteilen des Natriumsalzes der Diisobutylnaphtalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

VI. 3 Gewichtsteile der Verbindung des Beispiels 2 werden mit 97 Gewichtsteilen feinteiligem Kaolin innig vermischt. Man erhält auf diese Weise ein Stäubemittel, das 3 Gew.-% des Wirkstoffs enthält.

VII. 30 Gewichtsteile der Verbindung des Beispiels 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung des Beispiels 3 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wässerige Dispersion. Durch Verdünnen mit Wasser erhält man eine verdünnte wässerige Dispersion.

IX. 20 Teile der Verbindung des Beispiels 2 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die erfindungsgemässen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrösserung des fungiziden Wirkungsspektrums.

Die folgende Liste von Fungiziden, mit denen die erfindungsgemässen Verbindungen kombiniert werden können, soll die Kombinationsmöglichkeiten erläutern, nicht aber einschränken.

Fungizide, die mit den erfindungsgemässen Verbindungen kombiniert werden können, sind beispielsweise:

Schwefel,
Dithiocarbamate und deren Derivate, wie
Ferridimethyldithiocarbamat,
Zinkdimethyldithiocarbamat,
Mangan-Zink-ethylendiamin-bis-dithiocarbamat und
Zinkethylenbisdithiocarbamat,
Tetramethylthiuramdisulfide,
Ammoniak-Komplex von Zink-(N,N-ethylen-bis-dithiocarbamat) und
N,N'-Polyethylen-bis-(thiocarbamoyl)-disulfid,
Zink-(N,N'-propylen-bis-dithiocarbamat),
Ammoniak-Komplex von Zink-(N,N'-propylen-bis-dithiocarbamat) und
N,N'-Polypropylen-bis-(thiocarbamoyl)-disulfid;
Nitroderivate, wie
Dinitro-(1-methylheptyl)phenylcrotonat,
2-sec.-Butyl-4,5-dinitrophenyl-3,3-dimethylacrylat
2-sec.-Butyl-4,6-dinitrophenyl-isopropylcarbonat;
heterocyclische Substanzen, wie
N-(1,1,2,2-Tetrachlorethylthio)-tetrahydrophthalimid,
N-Trichlormethylthio-tetrahydrophthalimid,
2-Heptadecyl-2-imidazolin-acetat
2,4-Dichlor-6-(o-chloranilino)-s-triazin
0,0-Diethyl-phthalimidophonothioat
5-Amino-1-(bis-(dimethylamino)-phosphinyl)-3-phenyl-1,2,4-triazol
2,3-Dicyano-1,4-Dithioanthrachinon
2-Thio-1,3-dithio-(4,5-b)-chinoxalin
1-(Butylcarbamoyl)-2-benzimidazol-carbaminsäuremethylester
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin-4,4-dioxid
2,3-Dihydro-5-carboxanilido-6-methyl-1,4-oxathiin
2-(Furyl-(2))-benzimidazol
Piperazin-1,4-diylbis-1-(2,2,2-trichlor-ethyl)-formamid
2-Thiazolyl-(4)-benimidazol
6-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol
sowie verschiedene Fungizide, wie
Dodecylguanidinacetat
3-(3-(3,5-Dimethyl-2-oxycyclohexyl)-2-hydroxyethyl)-glutarimid
Hexachlorbenzol
N-Dichlorfluormethylthio-N',N'-dimethyl-N-phenyl-schwefelsäurediamid
2,5-Dimethyl-furan-3-carbonsäureanilid
2-Methyl-benzoesäure-anilid
2-Jod-benzoesäure-anilid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
DL-Methyl-N-(2,6-dimethyl-phenyl)-N-furoyl-(2)-alaninat,
DL-N-(2,6-Dimethyl-phenyl)-N-(2'-methoxyacetyl)-alaninmethylester,

5-Nitro-isophthalsäure-di-isopropylester,
1-(1',2',4'-Triazolyl-1')-[1-(4'-chlorphenoxy)]-3,3-dimethylbutan-2-on,
1-(1',2',4'-Triazolyl-1')-[1-(4'-chlorphenoxy)]-3,3-dimethylbutan-2-ol,
N-(2,6-Dimethylphenyl)-N-chloracetyl-D,L-2-aminobutyrolacton,
N-(n-Propyl)-N-(2,4,6-trichlorphenoxyethyl)-N'-imidazolylharnstoff,
N-Cyclohexyl-N-methoxy-2,5-dimethyl-furan-3-carbonsäureamid,
2,4,5-Trimethyl-furan-3-carbonsäureanilid,
5-Methyl-5-vinyl-3-(3,5-dichlorphenyl)-2,4-dioxo-1,3-oxazolidin,
5-Methoxymethyl-5-methyl-3-(3,5-dichlorphenyl)-2,4-dioxo-1,3-oxazolidin,
N-[3-(p-tert.-Butylphenyl)-2-methyl-propyl]-cis-2,6-dimethylmorpholin
N-Formyl-N-morpholin-2,2,2-trichlorethylacetal
1-[2-(2,4-Dichlorphenyl)-4-ethyl-1,3-dioxolan-2-ylmethyl]-1H-1,2,4-triazol
1-[2-(2,4-Dichlorphenyl)-4-n-propyl-1,3-dioxolan-2-yl-methyl]-1H-1,2,4-triazol

Für die folgenden Versuche wurde als bekannter Wirkstoff die Verbindung A verwendet (bekannt aus EP-A-39405)

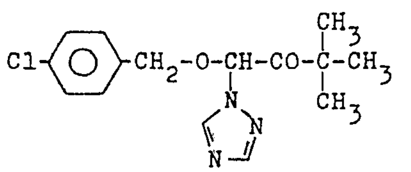
(A)

*Versuch 1*

Wirksamkeit gegen Weizenmehltau

Blätter von in Töpfen gewachsenen Weizenkeimlingen der Sorte «Jubilar» wurden mit wässeriger Spritzbrühe, die 80% Wirkstoff und 20% Emulgiermittel (Natrium-Ligninsulfonat) in der Trockensubstanz enthält, besprüht und 24 h nach dem Abtrocknen des Spritzbelages mit Oidien (Sporen) des Weizenmehltaus (Erysiphe graminis var. tritici) bestäubt. Die Versuchspflanzen wurden anschliessend im Gewächshaus bei Temperaturen zwischen 20 und 22°C und 75 bis 80% relativer Luftfeuchtigkeit aufgestellt. Nach 7 d wurde das Ausmass der Mehltauentwicklung ermittelt.

Das Ergebnis des Versuchs zeigte, dass beispielsweise die Wirkstoffe Nrn. 2, 9, 11, 13, 28, 30 bei Anwendung als 0,025, 0,006 oder 0,0015 %ige Wirkstoffbrühe eine bessere fungizide Wirksamkeit (beispielsweise 95%) zeigten als der Wirkstoff A (beispielsweise 60%).

*Versuch 2*

Wirksamkeit gegen Weizenbraunrost

Blätter von in Töpfen gewachsenen Weizensämlingen der Sorte «Jubilar» wurden mit Sporen des Braunrostes (Puccinia recondita) bestäubt. Danach wurden die Töpfe für 24 h bei 20 bis 22°C in eine Kammer mit hoher Luftfeuchtigkeit (90 bis 95%) gestellt. Während dieser Zeit keimen die Sporen aus und die Keimschläuche drangen in das Blattgewebe ein. Die infizierten Pflanzen wurden anschliessend mit wässerigen Spritzbrühen, die 80% Wirkstoff und 20% Emulgiermittel in der Trockensubstanz enthalten, tropfnass gespritzt. Nach dem Antrocknen des Spritzbelages wurden die Versuchspflanzen im Gewächshaus bei Temperaturen zwischen 20 und 22°C und 65 bis 70% relativer Luftfeuchte aufgestellt. Nach 8 d wurde das Ausmass der Rostpilzentwicklung auf den Blättern ermittelt.

Das Ergebnis des Versuchs zeigte, dass beispielsweise die Wirkstoffe Nrn. 2, 4, 5, 9, 13, 28, 29, 30 bei Anwendung als 0,025 oder 0,006%ige Wirkstoffbrühe eine bessere fungizide Wirksamkeit (beispielsweise 100%) zeigten als der Wirkstoff A (beispielsweise 45%).

*Versuch 3*

Wirksamkeit gegen Apfelschorf

Junge Blätter von in Töpfen gewachsenen Apfelsämlingen der Sorte «Golden Delicious» wurden mit wässeriger Spritzbrühe, die 80% Wirkstoff und 20% Emulgiermittel in der Trockensubstanz enthält, tropfnass gespritzt. Nach dem Antrocknen des Spritzbelages wurden die Versuchspflanzen mit einer Sporensuspension des Apfelschorfs (Venturia inaequalis) besprüht. Anschliessend wurden die inoculierten Pflanzen in einer Klimakammer bei 20 bis 22°C und 95% relativer Luftfeuchtigkeit für 10 d aufgestellt. Dann wurde das Ausmass der Pilzentwicklung auf den Blättern ermittelt.

Das Ergebnis des Versuchs zeigte, dass beispielsweise die Wirkstoffe Nrn. 4 und 5 bei Anwendung als 0,0075%ige Wirkstoffbrühe eine gut fungizide Wirksamkeit (beispielsweise 97%) zeigten.

**Patentansprüche** für die Vertragsstaaten BE, CH, DE, FR, GB, IT, LI, NL, SE

1. Triazolverbindungen der Formel (I)

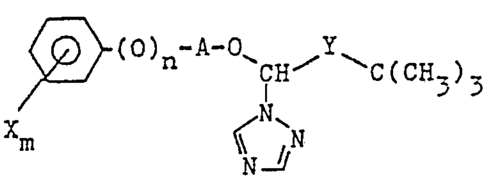
(I)

in welcher

X für Halogen, $C_1$-$C_4$-Alkyl, $C_1$-$C_4$-Alkyloxy, Trifluormethyl oder Phenyl steht,

m eine ganze Zahl von 0 bis 5 bedeutet, wobei die einzelnen Gruppen X gleich oder verschieden sind, wenn m grösser als 1 ist,

Y für CO oder die Gruppe $CR^1OR^2$ steht, in welcher $R^1$ Wasserstoff oder $C_1$-$C_4$-Alkyl bedeutet und $R^2$ Wasserstoff, $C_1$-$C_4$-Alkyl, $C_2$-$C_4$-Alkenyl oder $C_2$-$C_4$-Alkinyl bedeutet,

A für eine Alkylenkette mit 2 bis 6 Kohlenstoffatomen substituiert sein kann, und

n 0 oder 1 bedeutet,

sowie deren für Pflanzen verträgliche Säureadditionssalze und Metallkomplexe.

2. Triazolverbindungen der Formel (I) gemäss Anspruch 1, in welcher

X für Halogen steht,

Y für CO oder die Gruppe $CHOR^2$ steht, in welcher $R^2$ Wasserstoff, $C_1$-$C_4$-Alkyl oder $C_2$-$C_4$-Alkenyl bedeutet,

A für eine Alkylenkette mit 2 bis 6 Kohlenstoffatomen, die durch eine oder mehrere Methylgruppen substituiert sein kann, steht, und

n 0 oder 1 bedeutet.

3. Verfahren zur Herstellung von Azolverbindungen der Formel (I) gemäss Anspruch 1, dadurch gekennzeichnet, dass man

a) Halogenetherketone der Formel

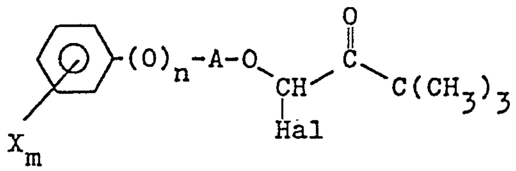

(II)

in welcher X, A, m und n die im Anspruch 1 angegebene Bedeutung haben und

Hal für Chlor oder Brom steht, mit 1,2,4-Triazol in Gegenwart eines Säurebindemittels und gegebenenfalls in Gegenwart eines Verdünnungsmittels umsetzt, oder

b) Triazolylhalogenketone der Formel

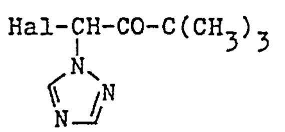

(III)

in welcher

Hal die oben angegebene Bedeutung hat, mit Alkoholen der Formel

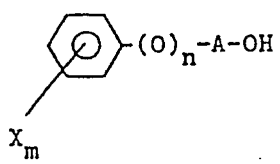

(IV)

in welcher X, A, m und n die oben angegebene Bedeutung haben, in Gegenwart eines Säurebindemittels und gegebenenfalls in Gegenwart eines Verdünnungsmittels umsetzt, und

c) gegebenenfalls die Keto-Derivate der Formel

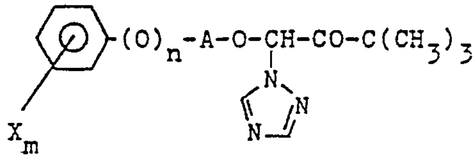

(Ia)

mit komplexen Hydriden oder mit Wasserstoff in Gegenwart von Hydrierungskatalysatoren reduziert oder mit Grignardreagenzien der Formel (V)

$R^1MgHal$ (V)

in der $R^1$ für $C_1$-$C_4$-Alkyl steht und in der Hal Chlor, Brom oder Iod bedeutet, umsetzt und die so erhaltenen Alkohole der Formel (I) ($Y=CR^1OH$) gegebenenfalls mit einem Alkylierungsmittel der Formel $L-R^2$, in der $R^2$ die oben genannten Bedeutungen hat und L Chlor oder Brom oder den Rest des Monoalkylschwefelsäureesters Alkyl$-OSO_2-$ bedeutet, alkyliert.

4. Fungizides Mittel, enthaltend eine Verbindung gemäss Anspruch 1.

5. Verfahren zur Bekämpfung von Fungi, dadurch gekennzeichnet, dass man eine Verbindung der Formel (I) gemäss Anspruch 1 auf Pilze oder durch Pilzbefall bedrohte Gegenstände, Flächen, Pflanzen oder Saatgüter einwirken lässt.

**Patentansprüche** für den Vertragsstaat: AT

1. Fungizid, enthaltend eine Triazolverbindung der Formel (I)

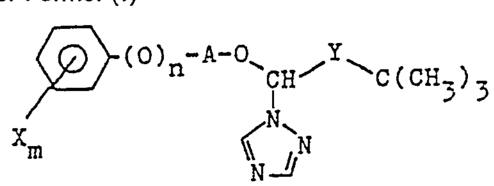

(I)

in welcher

X für Halogen, $C_1$-$C_4$-Alkyl, $C_1$-$C_4$-Alkyloxy, Trifluormethyl oder Phenyl steht,

m eine ganze Zahl von 0 bis 5 bedeutet, wobei die einzelnen Gruppen X gleich oder verschieden sind, wenn m grösser als 1 ist,

Y für CO oder die Gruppe $CR^1OR^2$ steht, in welcher $R^1$ Wasserstoff oder $C_1$-$C_4$-Alkyl bedeutet und $R^2$ Wasserstoff, $C_1$-$C_4$-Alkyl, $C_2$-$C_4$-Alkenyl oder $C_2$-$C_4$-Alkinyl bedeutet,

A für eine Alkylenkette mit 2 bis 6 Kohlenstoffatomen steht, die durch eine oder mehrere Alkylgruppen mit 1 bis 2 Kohlenstoffatomen substituiert sein kann, und

n 0 oder 1 bedeutet,

sowie deren für Pflanzen verträgliche Säureadditionssalze und Metallkomplexe.

2. Fungizid, enthaltend eine Triazolverbindung der Formel (I) gemäss Anspruch 1, in welcher

X für Halogen steht,

Y für CO oder die Gruppe $CHOR^2$ steht, in welcher $R^2$ Wasserstoff, $C_1$-$C_4$-Alkyl oder $C_2$-$C_4$-Alkenyl bedeutet,

A für eine Alkylenkette mit 2 bis 6 Kohlenstoffatomen, die durch eine oder mehrere Methylgruppen substituiert sein kann, steht, und

n 0 oder 1 bedeutet.

3. Verfahren zur Herstellung von Azolverbindungen der Formel (I) gemäss Anspruch 1, dadurch gekennzeichnet, dass man

a) Halogenetherketone der Formel

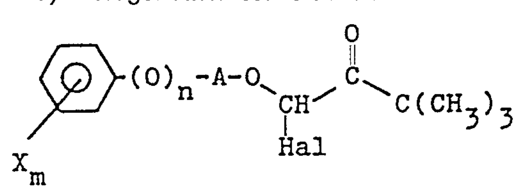

(II)

in welcher X, A, m und n die im Anspruch 1 angegebene Bedeutung haben und

Hal für Chlor oder Brom steht, mit 1,2,4-Triazol in Gegenwart eines Säurebindemittels und gegebenenfalls in Gegenwart eines Verdünnungsmittels umsetzt, oder

b) Triazolylhalogenketone der Formel

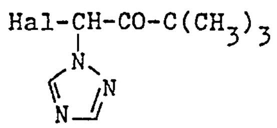 (III)

in welcher

Hal die oben angegebene Bedeutung hat, mit Alkoholen der Formel

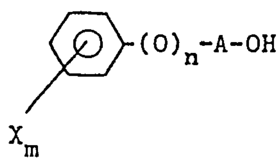 (IV)

in welcher X, A, m und n die oben angegebene Bedeutung haben, in Gegenwart eines Säurebindemittels und gegebenenfalls in Gegenwart eines Verdünnungsmittels umsetzt, und

c) gegebenenfalls die Keto-Derivate der Formel

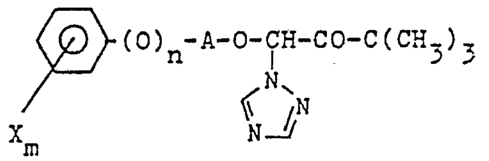 (Ia)

mit komplexen Hydriden oder mit Wasserstoff in Gegenwart von Hydrierungskatalysatoren reduziert oder mit Grignardreagenzien der Formel (V)

$$R^1MgHal \qquad (V)$$

in der $R^1$ für $C_1$-$C_4$-Alkyl steht und in der Hal Chlor, Brom oder Iod bedeutet, umsetzt und die so erhaltenen Alkohole der Formel (I) ($Y=CR^1OH$) gegebenenfalls mit einem Alkylierungsmittel der Formel $L-R^2$, in der $R^2$ die oben genannten Bedeutungen hat und L Chlor oder Brom oder den Rest des Monoalkylschwefelsäureesters $Alkyl-OSO_2-$ bedeutet, alkyliert.

4. Verfahren zur Bekämpfung von Fungi, dadurch gekennzeichnet, dass man eine Verbindung der Formel (I) gemäss Anspruch 1 auf Pilze oder durch Pilzbefall bedrohte Gegenstände, Flächen, Pflanzen oder Saatgüter einwirken lässt.

**Claims** for the Contracting States BE, CH, DE, FR, GB, IT, LI, NL, SE

1. A triazole compound of the formula (I)

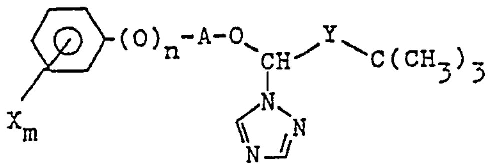 (I)

where

X is halogen, $C_1$-$C_4$-alkyl, $C_1$-$C_4$-alkoxy, trifluoromethyl or phenyl,

m is an integer from 0 to 5, the individual X's being identical or different when m is larger than 1,

Y is CO or $CR^1OR^2$, where $R^1$ is hydrogen or $C_1$-$C_4$-alkyl and $R^2$ is hydrogen, $C_1$-$C_4$-alkyl, $C_2$-$C_4$-alkenyl or $C_2$-$C_4$-alkynyl,

A is an alkylene chain which has 2 to 6 carbon atoms and is optionally substituted by one or more alkyl groups of 1 or 2 carbon atoms, and

n is 0 or 1,

and its plant-tolerated acid addition salts and metal complexes.

2. A triazole compound of the formula (I) as claimed in claim 1, where

X is halogen,

Y is CO or $CHOR^2$, where $R^2$ is hydrogen, $C_1$-$C_4$-alkyl or $C_2$-$C_4$-alkenyl,

A is an alkylene chain which has 2 to 6 carbon atoms and is optionally substituted by one or more methyl groups, and

n is 0 or 1.

3. A process for the preparation of an azole compound of the formula (I) as claimed in claim 1, wherein

(*a*) a haloether ketone of the formula

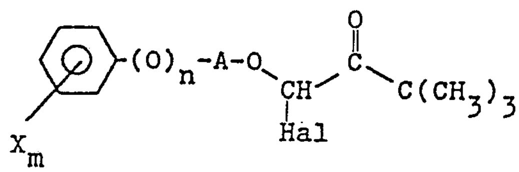 (II)

where X, A, m and n have the meanings given in claim 1, and Hal is chlorine or bromine, is reacted with 1,2,4-triazole in the presence of an acid acceptor and in the presence or absence of a diluent, or

(*b*) a triazolyl haloketone of the formula

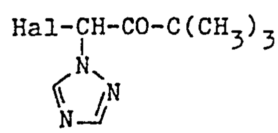 (III)

where Hal has the above meaning, is reacted with an alcohol of the formula

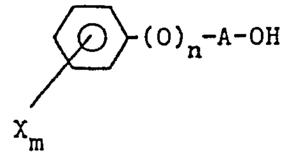 (IV)

where X, A, m and n have the above meanings, in the presence of an acid acceptor and in the presence or absence of a diluent, and

(*c*) if desired, the keto derivative of the formula

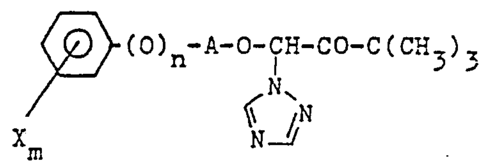 (Ia)

is reduced with a complex hydride or with hydrogen in the presence of a hydrogenation catalyst, or is reacted with a Grignard reagent of the formula (V)

$R^1MgHal$ (V)

where $R^1$ is $C_1$-$C_4$-alkyl and Hal is chlorine, bromine or iodine, and the resulting alcohol of the formula (I) ($Y=CR^1OH$) is optionally alkylated with an alkylating agent of the formula $L-R^2$, where $R^2$ has the above meanings and L is chlorine, bromine or the radical of the monoalkyl sulphate alkyl$-OSO_2-$.

4. A fungicidal agent containing a compound as claimed in claim 1.

5. A process for combating fungi, wherein a compound of the formula (I) as claimed in claim 1 is allowed to act on the fungi or the objects, areas, plants or seed threatened by fungal attack.

**Claims** for the Contracting State: AT

1. A fungicide containing a triazole compound of the formula (I)

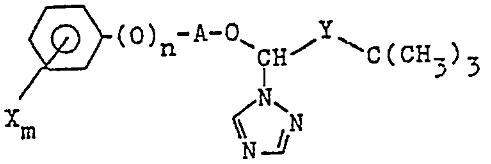 (I)

where

X is halogen, $C_1$-$C_4$-alkyl, $C_1$-$C_4$-alkoxy, trifluoromethyl or phenyl,

m is an integer from 0 to 5, the individual X's being identical or different when m is larger than 1,

Y is CO or $CR^1OR^2$, where $R^1$ is hydrogen or $C_1$-$C_4$-alkyl and $R^2$ is hydrogen, $C_1$-$C_4$-alkyl, $C_2$-$C_4$-alkenyl or $C_2$-$C_4$-alkynyl,

A is an alkylene chain which has 2 to 6 carbon atoms and is optionally substituted by one or more alkyl groups of 1 or 2 carbon atoms, and

n is 0 or 1,

and its plant-tolerated acid addition salts and metal complexes.

2. A fungicide containing a triazole compound of the formula (I) as claimed in claim 1, where

X is halogen,

Y is CO or $CHOR^2$, where $R^2$ is hydrogen, $C_1$-$C_4$-alkyl or $C_2$-$C_4$-alkenyl,

A is an alkylene chain which has 2 to 6 carbon atoms and is optionally substituted by one or more methyl groups, and

n is 0 or 1.

3. A process for the preparation of an azole compound of the formula (I) as claimed in claim 1, wherein

(*a*) a haloether ketone of the formula

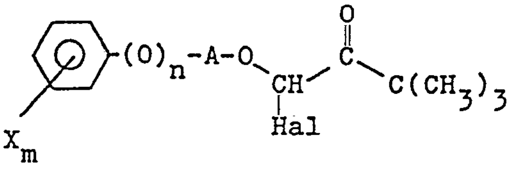 (II)

where X, A, m and n have the meanings given in claim 1, and Hal is chlorine or bromine, is reacted with 1,2,4-triazole in the presence of an acid acceptor and in the presence or absence of a diluent, or

(*b*) a triazolyl haloketone of the formula

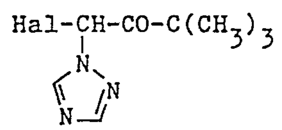 (III)

where Hal has the above meaning, is reacted with an alcohol of the formula

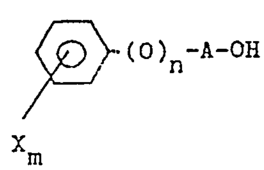 (IV)

where X, A, m and n have the above meanings, in the presence of an acid acceptor and in the presence or absence of a diluent, and

(*c*) if desired, the keto derivative of the formula

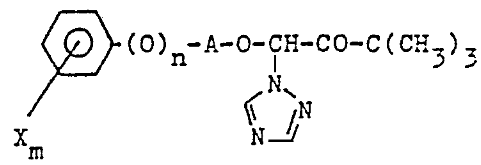 (Ia)

is reduced with a complex hydride or with hydrogen in the presence of a hydrogenation catalyst, or is reacted with a Grignard reagent of the formula (V)

$R^1MgHal$ (V)

where $R^1$ is $C_1$-$C_4$-alkyl and Hal is chlorine, bromine or iodine, and the resulting alcohol of the formula (I) ($Y=CR^1OH$) is optionally alkylated with an alkylating agent of the formula $L-R^2$, where $R^2$ has the above meanings and L is chlorine, bromine or the radical of the monoalkyl sulphate alkyl$-OSO_2-$.

4. A process for combating fungi, wherein a compound of the formula (I) as claimed in claim 1 is allowed to act on the fungi or the objects, areas, plants or seed threatened by fungal attack.

**Revendications** pour les Etats contractants BE, CH, DE, FR, GB, IT, LI, NL, SE

1. Dérivés du triazole de la formule (I)

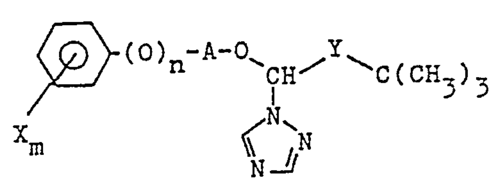 (I)

dans laquelle

X représente un atome d'halogène, un radical alkyle en $C_1$ à $C_4$ ou alkyl (en $C_1$ à $C_4$)-oxy ou un

groupe trifluoro-méthyle ou phényle, les divers substituants X pouvant être identiques ou différents, si

m, un nombre entier pouvant valoir 0 à 5, est supérieur à 1,

Y désigne un groupe CO ou un groupe $CR^1OR^2$, dans lequel $R^1$ peut désigner un atome d'hydrogène ou un radical alkyle en $C_1$ à $C_4$ et $R_2$ un atome d'hydrogène ou un radical alkyle en $C_1$ à $C_4$ ou alcényle en $C_2$ à $C_4$ ou alcynyle en $C_2$ à $C_4$,

A est un pont alkylène en $C_2$ à $C_6$, éventuellement substitué par un ou plusieurs radicaux alkyle en $C_1$ ou $C_2$ et

n vaut 0 ou 1,

ainsi que les sels d'addition d'acides et complexes de métaux compatibles avec les plantes de ces dérivés.

2. Dérivés du triazole de la formule (I) selon la revendication 1, dans laquelle

X désigne un atome d'halogène,

Y représente un groupe CO ou un groupe $CHOR^2$, où $R^2$ peut être un atome d'hydrogène ou un radical alkyle en $C_1$ à $C_4$ ou alcényle en $C_2$ à $C_4$,

A est un pont alkylène en $C_2$ à $C_6$, pouvant être substitué par un ou plusieurs radicaux méthyle et

n vaut 0 ou 1.

3. Procédé de préparation de composés azoliques de la formule (I) selon la revendication 1, caractérisé en ce que l'on fait réagir:

a) des éther-cétones halogénées de la formule

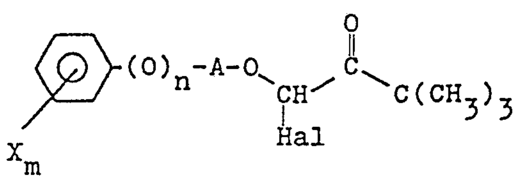 (II)

dans laquelle X, A, m et n possèdent la signification définie dans la revendication 1 et Hal désigne un atome de chlore ou de brome, en présence d'un agent fixant les acides et éventuellement dans un diluant, avec le 1,2,4-triazole; ou

b) des triazolyl-halo-cétones de la formule

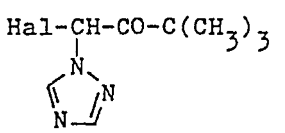 (III)

dans laquelle Hal possède la signification définie, en présence d'un agent fixant les acides et éventuellement dans un diluant, avec un alcool de la formule

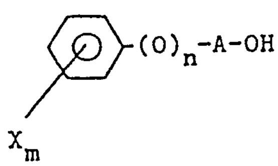 (IV)

dans laquelle X, A, m et n possèdent la signification définie plus haut, et on soumet éventuellement

c) les céto-dérivés de la formule

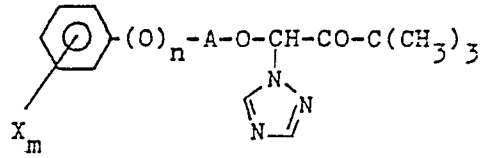 (Ia)

à une réduction par des hydrures complexes et l'hydrogène en présence d'un catalyseur d'hydrogénation ou à une réaction avec un réactif de Grignard de la formule

$$R^1MgHal \qquad (V)$$

dans laquelle $R^1$ désigne un radical alkyle en $C_1$ à $C_4$ et Hal un atome de chlore, de brome ou d'iode, les alcools obtenus, de la formule (I) avec $Y=CR^1OH$, étant éventuellement soumis à une alkylation à l'aide d'un agent d'alkylation de la formule $L-R^2$, dans laquelle $R^2$ possède l'une des significations définies plus haut et L désigne un atome de chlore ou de brome ou le reste d'un ester mono-alkylique de l'acide sulfurique $OSO_2$-alkyle.

4. Composition fongicide, contenant un composé selon la revendication 1.

5. Procédé pour combattre les champignons, caractérisé en ce que l'on fait agir un composé de la formule (I) selon la revendication 1 sur les champignons ou sur les objets, surfaces, plantes ou semences susceptibles d'être attaqués par des champignons.

**Revendications** pour l'Etat contractant: AT

1. Composition fongicide, contenant un dérivé du triazole de la formule (I)

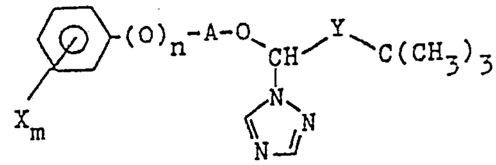 (I)

dans laquelle

X représente un atome d'halogène, un radical alkyle en $C_1$ à $C_4$ ou alkyl (en $C_1$ à $C_4$)-oxy ou un groupe trifluoro-méthyle ou phényle, les divers substituants X pouvant être identiques ou différents, si

m, un nombre entier pouvant valoir 0 à 5, est supérieur à 1,

Y désigne un groupe CO ou un groupe $CR^1OR^2$, dans lequel $R^1$ peut désigner un atome d'hydrogène ou un radical alkyle en $C_1$ à $C_4$ et $R^2$ un atome d'hydrogène ou un radical alkyle en $C_1$ à $C_4$ ou alcényle en $C_2$ à $C_4$ ou alcynyle en $C_2$ à $C_4$,

A est un pont alkylène en $C_2$ à $C_6$, éventuellement substitué par un ou plusieurs radicaux alkyle en $C_1$ ou $C_2$ et

n vaut 0 ou 1,

ou un sel d'addition d'acide ou complexe de métal compatible avec les plantes d'un tel dérivé.

2. Composition fongicide,contenant un dérivé du triazole de la formule (I) selon la revendication 1, pour lequel

X désigne un atome d'halogène,

Y représente un groupe CO ou un groupe $CHOR^2$, où $R^2$ peut être un atome d'hydrogène ou un radical alkyle en $C_1$ à $C_4$ ou alcényle en $C_2$ à $C_4$, A est un pont alkylène en $C_2$ à $C_6$, pouvant être substitué par un ou plusieurs radicaux méthyle et n vaut 0 ou 1.

3. Procédé de préparation de composés azoliques de la formule (I) selon la revendication 1, caractérisé en ce que l'on fait réagir:

a) des éther-cétones halogénées de la formule

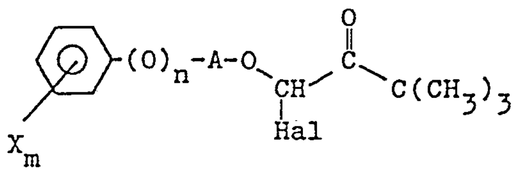
(II)

dans laquelle X, A, m et n possèdent la signification définie dans la revendication 1 et Hal désigne un atome de chlore ou de brome, en présence d'un agent fixant les acides et éventuellement dans un diluant, avec le 1,2,4-triazole; ou

b) des triazolyl-halo-cétones de la formule

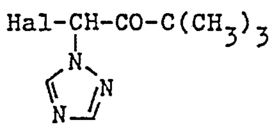
(III)

dans laquelle Hal possède la signification définie, en présence d'un agent fixant les acides et éventuellement dans un diluant, avec un alcool de la formule

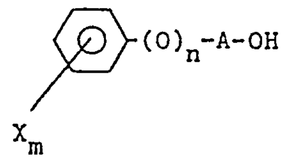
(IV)

dans laquelle X, A, m et n possèdent la signification définie plus haut, et on soumet éventuellement

c) les céto-dérivés de la formule

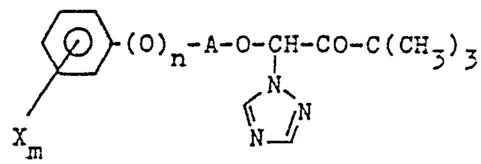
(Ia)

à une réduction par des hydrures complexes et l'hydrogène en présence d'un catalyseur d'hydrogénation ou à une réaction avec un réactif de Grignard de la formule

$$R^1MgHal \qquad (V)$$

dans laquelle $R^1$ désigne un radical alkyle en $C_1$ à $C_4$ et Hal un atome de chlore, de brome ou d'iode, les alcools obtenus, de la formule (I) avec $Y=CR^1OH$, étant éventuellement soumis à une alkylation à l'aide d'un agent d'alkylation de la formule $L-R^2$, dans laquelle $R^2$ possède l'une des significations définies plus haut et L désigne un atome de chlore ou de brome ou le reste d'un ester mono-alkylique de l'acide sulfurique $OSO_2-$alkyle.

4. Procédé pour combattre les champignons, caractérisé en ce que l'on fait agir un composé de la formule (I) selon la revendication 1, sur les champignons ou sur les objets, surfaces, plantes ou semences susceptibles d'être attaqués par des champignons.